(19) **Europäisches Patentamt / European Patent Office / Office européen des brevets**

(11) **EP 3 682 798 B1**

(12) **EUROPEAN PATENT SPECIFICATION**

(45) Date of publication and mention
of the grant of the patent:
**21.02.2024 Bulletin 2024/08**

(21) Application number: **19214855.9**

(22) Date of filing: **10.02.2016**

(51) International Patent Classification (IPC):
**A61B 5/352** (2021.01)

(52) Cooperative Patent Classification (CPC):
**A61N 1/36139; A61B 5/02405; A61B 5/0245;
A61B 5/1118; A61B 5/352; A61B 5/4848;
A61B 5/686; A61B 5/7275; A61N 1/36053;
A61N 1/36114; G16H 40/63;** A61B 5/363;
A61B 5/6877; A61B 2505/09

(54) **TITRATION OF HEART RATE TO IMPROVE STIMULATION THERAPY FOR HEART FAILURE PATIENTS**

HERZRATENTITRATION ZUR VERBESSERUNG DER STIMULATIONSTHERAPIE FÜR PATIENTEN MIT HERZINSUFFIZIENZ

TITRATION DE LA FRÉQUENCE CARDIAQUE POUR AMÉLIORER LA THÉRAPIE DE STIMULATION CHEZ DES PATIENTS SOUFFRANT D'INSUFFISANCE CARDIAQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **10.02.2015 US 201562114480 P**

(43) Date of publication of application:
**22.07.2020 Bulletin 2020/30**

(62) Document number(s) of the earlier application(s) in
accordance with Art. 76 EPC:
**16749776.7 / 3 256 208**

(73) Proprietor: **LivaNova USA, Inc.
Houston, TX 77058 (US)**

(72) Inventors:
• **CARLSON, Gerrard Merrill
Houston, TX Texas 77058 (US)**
• **SABESAN, Shivkumar
Houston, TX Texas 77058 (US)**
• **KENKNIGHT, Bruce H.
Houston, TX Texas 77058 (US)**
• **LIBBUS, Imad
Houston, TX Texas 77058 (US)**
• **AMURTHUR, Badri
Houston, TX Texas 77058 (US)**

(74) Representative: **Grünecker Patent- und
Rechtsanwälte
PartG mbB
Leopoldstraße 4
80802 München (DE)**

(56) References cited:
WO-A1-2015/148142     WO-A1-2016/025265
WO-A1-2016/025269     WO-A1-2016/077481
WO-A2-2014/039569     US-A1- 2010 010 556
US-A1- 2012 095 530   US-A1- 2013 238 047

## Description

CROSS-REFERENCE TO RELATED APPLICATIONS

The present application claims the benefit of U.S. Provisional Application No. 62/114,480, filed on February 10, 2015.

FIELD

This application relates to neuromodulation and, more specifically, to improved systems and methods for delivering stimulation therapies.

BACKGROUND

Chronic heart failure (CHF) and other forms of chronic cardiac dysfunction (CCD) may be related to an autonomic imbalance of the sympathetic and parasympathetic nervous systems that, if left untreated, can lead to cardiac arrhythmogenesis, progressively worsening cardiac function and eventual patient death. CHF is pathologically characterized by an elevated neuroexitatory state and is accompanied by physiological indications of impaired arterial and cardiopulmonary baroreflex function with reduced vagal activity.

CHF triggers compensatory activations of the sympathoadrenal (sympathetic) nervous system and the renin-angiotensin-aldosterone hormonal system, which initially helps to compensate for deteriorating heart-pumping function, yet, over time, can promote progressive left ventricular dysfunction and deleterious cardiac remodeling. Patients suffering from CHF are at increased risk of tachyarrhythmias, such as atrial fibrillation (AF), ventricular tachyarrhythmias (ventricular tachycardia (VT) and ventricular fibrillation (VF)), and atrial flutter, particularly when the underlying morbidity is a form of coronary artery disease, cardiomyopathy, mitral valve prolapse, or other valvular heart disease. Sympathoadrenal activation also significantly increases the risk and severity of tachyarrhythmias due to neuronal action of the sympathetic nerve fibers in, on, or around the heart and through the release of epinephrine (adrenaline), which can exacerbate an already-elevated heart rate.

The standard of care for managing CCD in general continues to evolve. For instance, new therapeutic approaches that employ electrical stimulation of neural structures that directly address the underlying cardiac autonomic nervous system imbalance and dysregulation have been proposed. In one form, controlled stimulation of the cervical vagus nerve beneficially modulates cardiovascular regulatory function. Vagus nerve stimulation (VNS) has been used for the clinical treatment of drug-refractory epilepsy and depression, and more recently has been proposed as a therapeutic treatment of heart conditions such as CHF. A system for neurostimulation is disclosed in US 2012/095530.

One challenge with the use of VNS for the treatment of heart conditions is that it is difficult to identify objective measures for determining the efficacy of the treatment. In particular, it would be desirable to identify a physiological signal that could be used as an objective metric for evaluating and/or improving treatment.

SUMMARY

Systems and methods are provided for delivering neurostimulation therapies to patients. The patient's intrinsic heart rate recovery may be used to determine efficacy of therapy and to adjust stimulation parameters.

A method, which is not part of the claimed invention, of operating an implantable medical device (IMD) comprising a neurostimulator coupled to an electrode assembly is provided. The method comprises: monitoring a pre-therapy heart rate recovery (HRR) rate of a patient prior to initiation of a stimulation therapy; initiating the stimulation therapy; monitoring the post-therapy HRR rate of the patient after initiation of the stimulation therapy; and comparing the pre-therapy HRR rate to the post-therapy HRR rate.

A method, which is not part of the claimed invention, of operating an implantable medical device (IMD) comprising a neurostimulator coupled to an electrode assembly is provided. The method comprises: initiating a stimulation therapy with the neurostimulator; monitoring an activity level of the patient; and modifying one or more stimulation parameters of the stimulation therapy in response to monitored changes in the activity level of the patient.

The claims define the matter for which protection is sought in terms of the technical features of the invention.

BRIEF DESCRIPTION OF DRAWINGS

FIG. 1 is a front anatomical diagram showing, by way of example, placement of an implantable vagus stimulation device in a male patient.
FIGS. 2A and 2B are diagrams respectively showing the implantable neurostimulator and the simulation therapy lead of FIG. 1.
FIG. 3 is a diagram showing an external programmer for use with the implantable neurostimulator of FIG. 1.
FIG. 4 is a diagram showing electrodes provided as on the stimulation therapy lead of FIG. 2 in place on a vagus nerve in situ.
FIG. 5 is a graph showing, by way of example, the relationship between the targeted therapeutic efficacy and the extent of potential side effects resulting from use of the implantable neurostimulator of FIG. 1.
FIG. 6 is a graph showing, by way of example, the optimal duty cycle range based on the intersection depicted in FIG. 5.

FIG. 7 is a timing diagram showing, by way of example, a stimulation cycle and an inhibition cycle of VNS as provided by implantable neurostimulator of FIG. 1.

FIGS. 8A-8C are illustrative charts reflecting a heart rate response to gradually increased stimulation intensity at different frequencies.

FIGS. 9A-9B are block diagrams of neurostimulation systems in accordance with embodiments of the present invention.

FIGS. 10A-10B are charts illustrating extracting an intrinsic heart rate recovery parameter, in accordance with embodiments of the present invention.

FIG. 11 is a flowchart illustrating a method of identifying IHRR events from smoothed heart rate data

FIG. 12 is a histogram of relaxation parameters plotted against corresponding changes in heart rate for a single patient over a certain period of time.

FIG. 13 is a line chart of the mean banded relaxation parameter as a function of the corresponding change in HR.

FIG. 14 illustrates the mean banded relaxation parameters as a function of the corresponding change in HR for a subgroup of patients deemed to be non-responders to therapy.

FIG. 15 illustrates the mean banded relaxation parameters as a function of the corresponding change in HR for patients who were deemed to be responders to the therapy.

DETAILED DESCRIPTION

CHF and other cardiovascular diseases cause derangement of autonomic control of the cardiovascular system, favoring increased sympathetic and decreased parasympathetic central outflow. These changes are accompanied by elevation of basal heart rate arising from chronic sympathetic hyperactivation along the neurocardiac axis.

The vagus nerve is a diverse nerve trunk that contains both sympathetic and parasympathetic fibers, and both afferent and efferent fibers. These fibers have different diameters and myelination, and subsequently have different activation thresholds. This results in a graded response as intensity is increased. Low intensity stimulation results in a progressively greater tachycardia, which then diminishes and is replaced with a progressively greater bradycardia response as intensity is further increased. Peripheral neurostimulation therapies that target the fluctuations of the autonomic nervous system have been shown to improve clinical outcomes in some patients. Specifically, autonomic regulation therapy results in simultaneous creation and propagation of efferent and afferent action potentials within nerve fibers comprising the cervical vagus nerve. The therapy directly improves autonomic balance by engaging both medullary and cardiovascular reflex control components of the autonomic nervous system. Upon stimulation of the cervical vagus nerve, action potentials propagate away from the stimulation site in two directions, efferently toward the heart and afferently toward the brain. Efferent action potentials influence the intrinsic cardiac nervous system and the heart and other organ systems, while afferent action potentials influence central elements of the nervous system.

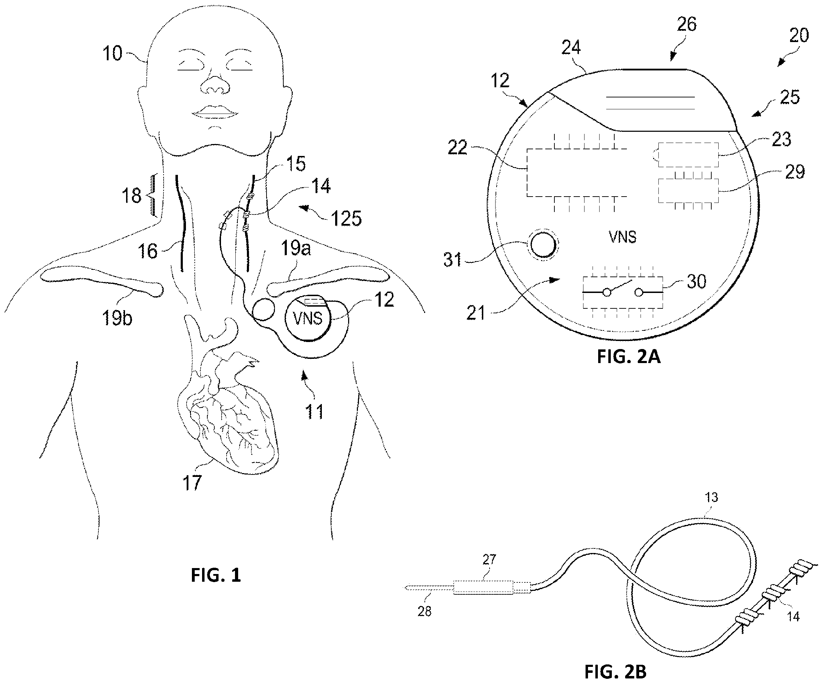

An implantable vagus nerve stimulator, such as used to treat drug-refractory epilepsy and depression, can be adapted for use in managing chronic cardiac dysfunction (CCD) through therapeutic bi-directional vagus nerve stimulation. FIG. 1 is a front anatomical diagram showing, by way of example, placement of an implantable medical device (e.g., a vagus nerve stimulation (VNS) system 11, as shown in FIG. 1) in a male patient 10. The VNS provided through the stimulation system 11 operates under several mechanisms of action. These mechanisms include increasing parasympathetic outflow and inhibiting sympathetic effects by inhibiting norepinephrine release and adrenergic receptor activation. More importantly, VNS triggers the release of the endogenous neurotransmitter acetylcholine and other peptidergic substances into the synaptic cleft, which has several beneficial anti-arrhythmic, anti-apoptotic, and anti-inflammatory effects as well as beneficial effects at the level of the central nervous system.

The implantable vagus stimulation system 11 comprises an implantable neurostimulator or pulse generator 12 and a stimulating nerve electrode assembly 125. The stimulating nerve electrode assembly 125, preferably comprising at least an electrode pair, is conductively connected to the distal end of an insulated, electrically conductive lead assembly 13 and electrodes 14. The electrodes 14 may be provided in a variety of forms, such as, e.g., helical electrodes, probe electrodes, cuff electrodes, as well as other types of electrodes.

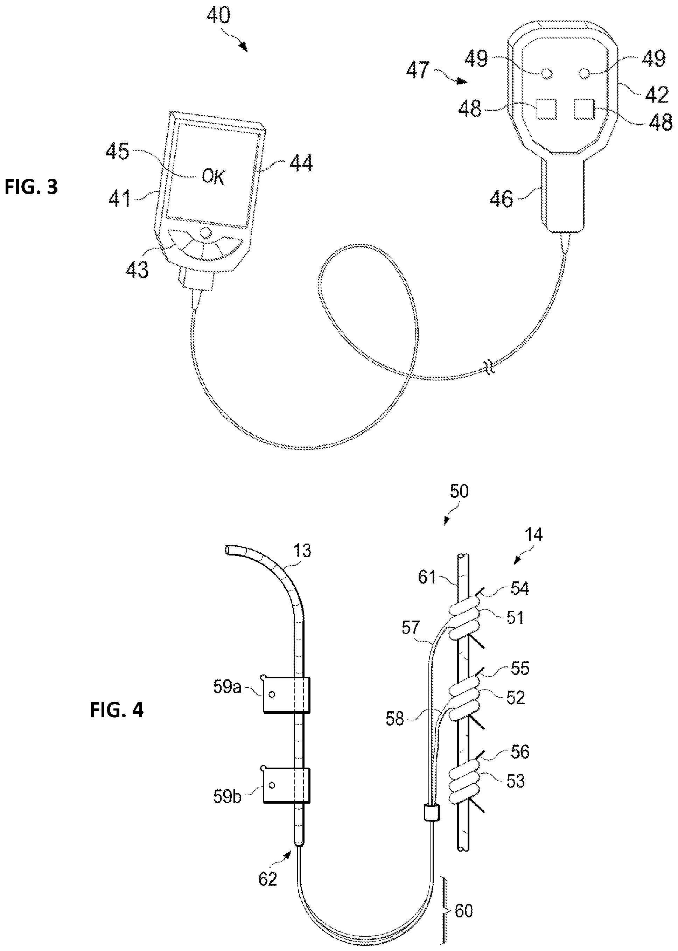

The implantable vagus stimulation system 11 can be remotely accessed following implant through an external programmer, such as the programmer 40 shown in FIG. 3 and described in further detail below. The programmer 40 can be used by healthcare professionals to check and program the neurostimulator 12 after implantation in the patient 10 and to adjust stimulation parameters during the initial stimulation titration process. Several classes of implantable medical devices provide therapy using electrical current as a stimulation vehicle. When such a system stimulates certain organs or body structures like the vagus nerve, therapeutic levels of electrical stimulation are usually not well tolerated by patients without undergoing a process known as titration. Titration is a systematic method of slowly increasing, over time, stimulation parameters employed by an implanted device to deliver stimulation current until therapeutic levels become tolerated by the patient.

An external magnet may provide basic controls, such as described in commonly assigned U.S. Patent No. 8,600,505, entitled "Implantable Device For Facilitating Control Of Electrical Stimulation Of Cervical Vagus Nerves For Treatment Of Chronic Cardiac Dysfunction". For further example, an electromagnetic controller may enable the patient 10 or healthcare professional to interact with the implanted neurostimulator 12 to exercise increased control over therapy delivery and suspension, such as described in commonly-assigned U.S. Patent No. 8,571,654, entitled "Vagus Nerve Neurostimulator With Multiple Patient-Selectable Modes For Treating Chronic Cardiac Dysfunction". For further example, an external programmer may communicate with the neurostimulation system 11 via other wired or wireless communication methods, such as, e.g., wireless RF transmission. Together, the implantable vagus stimulation system 11 and one or more of the external components form a VNS therapeutic delivery system.

The neurostimulator 12 is typically implanted in the patient's right or left pectoral region generally on the same side (ipsilateral) as the vagus nerve 15, 16 to be stimulated, although other neurostimulator-vagus nerve configurations, including contra-lateral and bi-lateral are possible. A vagus nerve typically comprises two branches that extend from the brain stem respectively down the left side and right side of the patient, as seen in FIG. 1. The electrodes 14 are generally implanted on the vagus nerve 15, 16 about halfway between the clavicle 19a-b and the mastoid process. The electrodes may be implanted on either the left or right side. The lead assembly 13 and electrodes 14 are implanted by first exposing the carotid sheath and chosen branch of the vagus nerve 15, 16 through a latero-cervical incision (perpendicular to the long axis of the spine) on the ipsilateral side of the patient's neck 18. The helical electrodes 14 are then placed onto the exposed nerve sheath and tethered. A subcutaneous tunnel is formed between the respective implantation sites of the neurostimulator 12 and helical electrodes 14, through which the lead assembly 13 is guided to the neurostimulator 12 and securely connected.

The neural stimulation may be provided as a low level maintenance dose independent of cardiac cycle. The stimulation system 11 bi-directionally stimulates either the left vagus nerve 15 or the right vagus nerve 16. However, it is contemplated that multiple electrodes 14 and multiple leads 13 could be utilized to stimulate simultaneously, alternatively or in other various combinations. Stimulation may be through multimodal application of continuously-cycling, intermittent and periodic electrical stimuli, which are parametrically defined through stored stimulation parameters and timing cycles. Both sympathetic and parasympathetic nerve fibers in the vagosympathetic complex are stimulated. A study of the relationship between cardiac autonomic nerve activity and blood pressure changes in ambulatory dogs is described in J. Hellyer et al., "Autonomic Nerve Activity and Blood Pressure in Ambulatory Dogs," Heart Rhythm, Vol. 11(2), pp. 307-313 (February 2014). Generally, cervical vagus nerve stimulation results in propagation of action potentials from the site of stimulation in a bi-directional manner. The application of bi-directional propagation in both afferent and efferent directions of action potentials within neuronal fibers comprising the cervical vagus nerve improves cardiac autonomic balance. Afferent action potentials propagate toward the parasympathetic nervous system's origin in the medulla in the nucleus ambiguus, nucleus tractus solitarius, and the dorsal motor nucleus, as well as towards the sympathetic nervous system's origin in the intermediolateral cell column of the spinal cord. Efferent action potentials propagate toward the heart 17 to activate the components of the heart's intrinsic nervous system. Either the left or right vagus nerve 15, 16 can be stimulated by the stimulation system 11. The right vagus nerve 16 has a moderately lower (approximately 30%) stimulation threshold than the left vagus nerve 15 for heart rate effects at the same stimulation frequency and pulse width.

The VNS therapy is delivered autonomously to the patient's vagus nerve 15, 16 through three implanted components that include a neurostimulator 12, lead assembly 13, and electrodes 14. FIGS. 2A and 2B are diagrams respectively showing the implantable neurostimulator 12 and the stimulation lead assembly 13 of FIG. 1. The neurostimulator 12 can be adapted from a VNS Therapy Demipulse Model 103 or AspireSR Model 106 pulse generator, manufactured and sold by LivaNova PLC, Houston, Texas, although other manufactures and types of implantable VNS neurostimulators could also be used. The stimulation lead assembly 13 and electrodes 14 are generally fabricated as a combined assembly and can be adapted from a Model 302 lead, PerenniaDURA Model 303 lead, or PerenniaFLEX Model 304 lead, also manufactured and sold by LivaNova PLC, in three sizes based, for example, on a helical electrode inner diameter, although other manufactures and types of single-pin receptacle-compatible therapy leads and electrodes could also be used.

Referring first to FIG. 2A, the system 20 may be configured to provide multimodal vagus nerve stimulation. In a maintenance mode, the neurostimulator 12 is parametrically programmed to deliver continuously-cycling, intermittent and periodic ON-OFF cycles of VNS. Such delivery produces action potentials in the underlying nerves that propagate bi-directionally, both afferently and efferently.

The neurostimulator 12 includes an electrical pulse generator that is tuned to improve autonomic regulatory function by triggering action potentials that propagate both afferently and efferently within the vagus nerve 15, 16. The neurostimulator 12 is enclosed in a hermetically sealed housing 21 constructed of a biocompatible material, such as titanium. The housing 21 contains electronic circuitry 22 powered by a battery 23, such as a lithium carbon monofluoride primary battery or a rechargeable secondary cell battery. The electronic circuitry 22 may be implemented using complementary metal oxide semiconductor integrated circuits that include a microprocessor controller that executes a control program according to stored stimulation parameters and timing cycles; a voltage regulator that regulates system power; logic and control circuitry, including a recordable memory 29 within which the stimulation parameters are stored, that controls overall pulse generator function, receives and implements programming commands from the external programmer, or other external source, collects and stores telemetry information, processes sensory input, and controls scheduled and sensory-based therapy outputs; a transceiver that remotely communicates with the external programmer using radio frequency signals; an antenna, which receives programming instructions and transmits the telemetry information to the external programmer; and a reed switch 30 that provides remote access to the operation of the neurostimulator 12 using an external programmer, a simple patient magnet, or an electromagnetic controller. The recordable memory 29 can include both volatile (dynamic) and non-volatile/persistent (static) forms of memory, within which the stimulation parameters and timing cycles can be stored. Other electronic circuitry and components are possible.

The neurostimulator 12 includes a header 24 to securely receive and connect to the lead assembly 13. The header 24 may enclose a receptacle 25 into which a single pin for the lead assembly 13 can be received, although two or more receptacles could also be provided, along with the corresponding electronic circuitry 22. The header 24 internally includes a lead connector block (not shown), a setscrew, and a spring contact (not shown) that electrically connects to the lead ring, thus completing the electrical circuit 26.

The housing 21 may also contain a heart rate sensor 31 that is electrically interfaced with the logic and control circuitry, which receives the patient's sensed heart rate as sensory inputs. The heart rate sensor 31 monitors heart rate using an electrocardiogram (ECG)-type electrode. Through the electrode, the patient's heart beat can be sensed by detecting ventricular depolarization. A plurality of electrodes can be used to sense voltage differentials between electrode pairs, which can undergo signal processing for cardiac physiological measures, for instance, detection of the P-wave, QRS complex, and T-wave. The heart rate sensor 31 provides the sensed heart rate to the control and logic circuitry as sensory inputs that can be used to determine the onset or presence of arrhythmias, particularly VT, and/or to monitor and record changes in the patient's heart rate over time or in response to applied stimulation signals.

Referring next to FIG. 2B, the lead assembly 13 delivers an electrical signal from the neurostimulator 12 to the vagus nerve 15, 16 via the electrodes 14. On a proximal end, the lead assembly 13 has a lead connector 27 that transitions an insulated electrical lead body to a metal connector pin 28 and metal connector ring. During implantation, the connector pin 28 is guided through the receptacle 25 into the header 24 and securely fastened in place using a setscrew (not shown) that engages the connector pin 28 to electrically couple one electrode of the lead assembly 13 to the neurostimulator 12 while the spring contact makes electrical contact to the ring connected to the other electrode. On a distal end, the lead assembly 13 terminates with the electrode 14, which bifurcates into a pair of anodic and cathodic electrodes 62 (as further described infra with reference to FIG. 4). The lead connector 27 may be manufactured using silicone and the connector pin 28 and ring are made of stainless steel, although other suitable materials could be used, as well. The insulated lead body 13 utilizes a silicone-insulated alloy conductor material.

The electrodes 14 may be helical and placed around the cervical vagus nerve 15, 16 at the location below where the superior and inferior cardiac branches separate from the cervical vagus nerve. Alternatively, the helical electrodes may be placed at a location above where one or both of the superior and inferior cardiac branches separate from the cervical vagus nerve. The helical electrodes 14 may be positioned around the patient's vagus nerve oriented with the end of the helical electrodes 14 facing the patient's head. Alternatively, the helical electrodes 14 may be positioned around the patient's vagus nerve 15, 16 oriented with the end of the helical electrodes 14 facing the patient's heart 17. At the distal end, the insulated electrical lead body 13 is bifurcated into a pair of lead bodies that are connected to a pair of electrodes. The polarity of the electrodes could be configured into a proximal anode and a distal cathode, or a proximal cathode and a distal anode.

The neurostimulator 12 may be interrogated prior to implantation and throughout the therapeutic period with a healthcare provider-operable control system comprising an external programmer and programming wand (shown in FIG. 3) for checking proper operation, downloading recorded data, diagnosing problems, and programming operational parameters, such as described in commonly-assigned U.S. Patent Nos. 8,600,505 and 8,571,654, cited supra. FIG. 3 is a diagram showing an external programmer 40 for use with the implantable neurostimulator 12 of FIG. 1. The external programmer 40 includes a healthcare provider operable programming computer 41 and a programming wand 42. Generally, use of the external programmer is restricted to healthcare providers, while more limited manual control is provided to the patient through "magnet mode."

The external programmer 40 may execute application software 45 specifically designed to interrogate the neurostimulator 12. The programming computer 41 interfaces to the programming wand 42 through a wired or wireless data connection. The programming wand 42 can be adapted from a Model 201 Programming Wand, manufactured and sold by LivaNova PLC, and the application software 45 can be adapted from the Model 250 Programming Software suite, licensed by LivaNova PLC. Other configurations and combinations of external programmer 40, programming wand 42 and application software 45 are possible.

The programming computer 41 can be implemented using a general purpose programmable computer and can be a personal computer, laptop computer, ultrabook computer, netbook computer, handheld computer, tablet computer, smart phone, or other form of computational device. The programming computer may be a tablet computer that may operate under the iOS operating system from Apple Inc., such as the iPad from Apple Inc., or may operate under the Android operating system from Google Inc., such as the Galaxy Tab from Samsung Electronics Co., Ltd. Alternatively, the programming computer may be a personal digital assistant handheld computer operating under the Pocket-PC, Windows Mobile, Windows Phone, Windows RT, or Windows operating systems, licensed by Microsoft Corporation, Redmond,Washington, such as the Surface from Microsoft Corporation, the Dell Axim X5 and X50 personal data assistants, sold by Dell, Inc., Round Top, Texas, the HP Jornada personal data assistant, sold by Hewlett-Packard Company, Palo Alto, California. The programming computer 41 functions through those components conventionally found in such devices, including, for instance, a central processing unit, volatile and persistent memory, touch-sensitive display, control buttons, peripheral input and output ports, and network interface. The computer 41 operates under the control of the application software 45, which is executed as program code as a series of process or method modules or steps by the programmed computer hardware. Other assemblages or configurations of computer hardware, firmware, and software are possible.

Operationally, the programming computer 41, when connected to a neurostimulator 12 through wireless telemetry using the programming wand 42, can be used by a healthcare provider to remotely interrogate the neurostimulator 12 and modify stored stimulation parameters. The programming wand 42 provides data conversion between the digital data accepted by and output from the programming computer and the radio frequency signal format that is required for communication with the neurostimulator 12. The programming computer may communicate with the implanted neurostimulator 12 using other wireless communication methods, such as wireless RF transmission. The programming computer 41 may further be configured to receive inputs, such as physiological signals received from patient sensors (e.g., implanted or external). These sensors may be configured to monitor one or more physiological signals, e.g., vital signs, such as body temperature, pulse rate, respiration rate, blood pressure, etc. These sensors may be coupled directly to the programming computer 41 or may be coupled to another instrument or computing device which receives the sensor input and transmits the input to the programming computer 41. The programming computer 41 may monitor, record, and/or respond to the physiological signals in order to effectuate stimulation delivery.

The healthcare provider operates the programming computer 41 through a user interface that includes a set of input controls 43 and a visual display 44, which could be touch-sensitive, upon which to monitor progress, view downloaded telemetry and recorded physiology, and review and modify programmable stimulation parameters. The telemetry can include reports on device history that provide patient identifier, implant date, model number, serial number, magnet activations, total ON time, total operating time, manufacturing date, and device settings and stimulation statistics and on device diagnostics that include patient identifier, model identifier, serial number, firmware build number, implant date, communication status, output current status, measured current delivered, lead impedance, and battery status. Other kinds of telemetry or telemetry reports are possible.

During interrogation, the programming wand 42 is held by its handle 46 and the bottom surface 47 of the programming wand 42 is placed on the patient's chest over the location of the implanted neurostimulator 12. A set of indicator lights 49 can assist with proper positioning of the wand and a set of input controls 48 enable the programming wand 42 to be operated directly, rather than requiring the healthcare provider to awkwardly coordinate physical wand manipulation with control inputs via the programming computer 41. The sending of programming instructions and receipt of telemetry information occur wirelessly through radio frequency signal interfacing. Other programming computer and programming wand operations are possible.

FIG. 4 is a diagram showing the helical electrodes 14 provided on the stimulation lead assembly 13 of FIG. 2 in place on a vagus nerve 15, 16 in situ 50. Although described with reference to a specific manner and orientation of implantation, the specific surgical approach and implantation site selection particulars may vary, depending upon physician discretion and patient physical structure.

Helical electrodes 14 may be positioned on the patient's vagus nerve 61 oriented with the end of the helical electrodes 14 facing the patient's head. At the distal end, the insulated electrical lead body 13 is bifurcated into a pair of lead bodies 57, 58 that are connected to a pair of electrodes 51, 52. The polarity of the electrodes 51, 52 could be configured into a proximal anode and a distal cathode, or a proximal cathode and a distal anode. In addition, an anchor tether 53 is fastened over the lead bodies 57, 58 that maintains the helical electrodes' position on the vagus nerve 61 following implant. The conductors of the electrodes 51, 52 may be manufactured using a platinum and iridium alloy, while the helical materials of the electrodes 51, 52 and the anchor tether 53 are a silicone elastomer.

During surgery, the electrodes 51, 52 and the anchor tether 53 are coiled around the vagus nerve 61 proximal to the patient's head, each with the assistance of a pair of sutures 54, 55, 56, made of polyester or other suitable material, which help the surgeon to spread apart the respective helices. The lead bodies 57, 58 of the electrodes 51, 52 are oriented distal to the patient's head and aligned parallel to each other and to the vagus nerve 61.

A strain relief bend 60 can be formed on the distal end with the insulated electrical lead body 13 aligned, for example, parallel to the helical electrodes 14 and attached to the adjacent fascia by a plurality of tie-downs 59a-b.

The neurostimulator 12 delivers VNS under control of the electronic circuitry 22. The stored stimulation parameters are programmable. Each stimulation parameter can be independently programmed to define the characteristics of the cycles of therapeutic stimulation and inhibition to ensure optimal stimulation for a patient 10. The programmable stimulation parameters include output current, signal frequency, pulse width, signal ON time, signal OFF time, magnet activation (for VNS specifically triggered by "magnet mode"), and reset parameters. Other programmable parameters are possible. In addition, sets or "profiles" of preselected stimulation parameters can be provided to physicians with the external programmer and fine-tuned to a patient's physiological requirements prior to being programmed into the neurostimulator 12, such as described in commonly-assigned U.S. Patent No. 8,630,709, entitled "Computer-Implemented System and Method for Selecting Therapy Profiles of Electrical Stimulation of Cervical Vagus Nerves for Treatment of Chronic Cardiac Dysfunction".

Therapeutically, the VNS may be delivered as a multimodal set of therapeutic doses, which are system output behaviors that are pre-specified within the neurostimulator 12 through the stored stimulation parameters and timing cycles implemented in firmware and executed by the microprocessor controller. The therapeutic doses include a maintenance dose that includes continuously-cycling, intermittent and periodic cycles of electrical stimulation during periods in which the pulse amplitude is greater than 0 mA ("therapy ON") and during periods in which the pulse amplitude is 0 mA ("therapy OFF").

The neurostimulator 12 can operate either with or without an integrated heart rate sensor, such as respectively described in commonly-assigned U.S. Patent No. 8,577,458, entitled "Implantable Device for Providing Electrical Stimulation of Cervical Vagus Nerves for Treatment of Chronic Cardiac Dysfunction with Leadless Heart Rate Monitoring," and U.S. Patent application, entitled "Implantable Device for Providing Electrical Stimulation of Cervical Vagus Nerves for Treatment of Chronic Cardiac Dysfunction," Serial No. 13/314,119, filed on December 7, 2011. Additionally, where an integrated leadless heart rate monitor is available, the neurostimulator 12 can provide autonomic cardiovascular drive evaluation and self-controlled titration, such as respectively described in commonly-assigned U.S. Patent No. 8,918,190, entitled "Implantable Device for Evaluating Autonomic Cardiovascular Drive in a Patient Suffering from Chronic Cardiac Dysfunction," filed on December 7, 2011, and U.S. Patent No. 8,918,191, entitled "Implantable Device for Providing Electrical Stimulation of Cervical Vagus Nerves for Treatment of Chronic Cardiac Dysfunction with Bounded Titration," filed on December 7, 2011. Finally, the neurostimulator 12 can be used to counter natural circadian sympathetic surge upon awakening and manage the risk of cardiac arrhythmias during or attendant to sleep, particularly sleep apneic episodes, such as respectively described in commonly-assigned U.S. Patent No. 8,923,964, entitled "Implantable Neurostimulator-Implemented Method For Enhancing Heart Failure Patient Awakening Through Vagus Nerve Stimulation," filed on November 9, 2012.

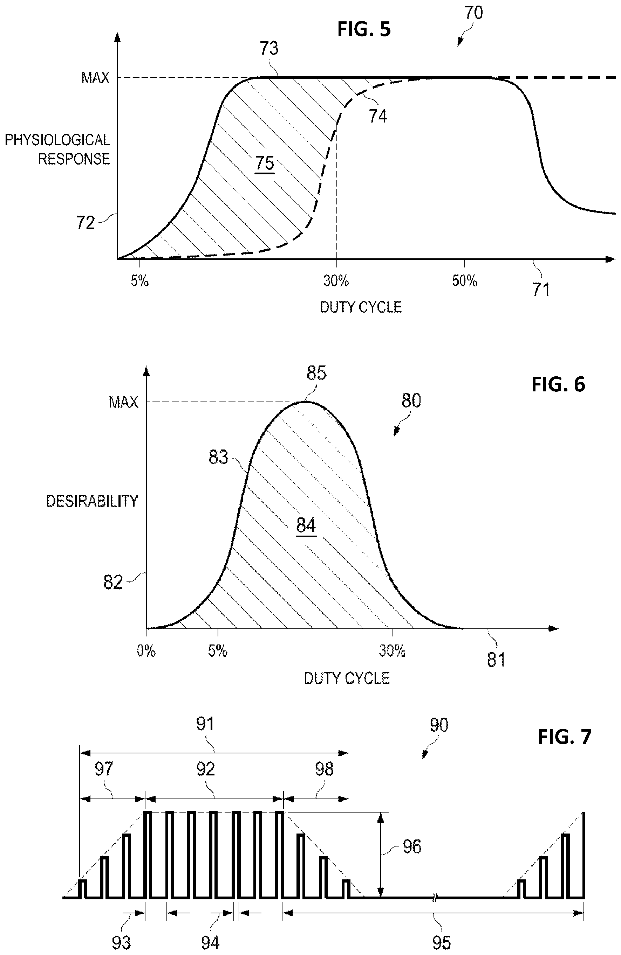

The VNS stimulation signal may be delivered as a therapy in a maintenance dose having an intensity that is insufficient to elicit undesirable side effects, such as cardiac arrhythmias. The VNS can be delivered with a periodic duty cycle in the range of 2% to 89% with a preferred range of around 4% to 36% that is delivered as a low intensity maintenance dose. Alternatively, the low intensity maintenance dose may comprise a narrow range approximately at 17.5%, such as around 15% to 25%. The selection of duty cycle is a tradeoff among competing medical considerations. The duty cycle is determined by dividing the stimulation ON time by the sum of the ON and OFF times of the neurostimulator 12 during a single ON-OFF cycle. However, the stimulation time may also need to include ramp-up time and ramp-down time, where the stimulation frequency exceeds a minimum threshold (as further described infra with reference to FIG. 7).

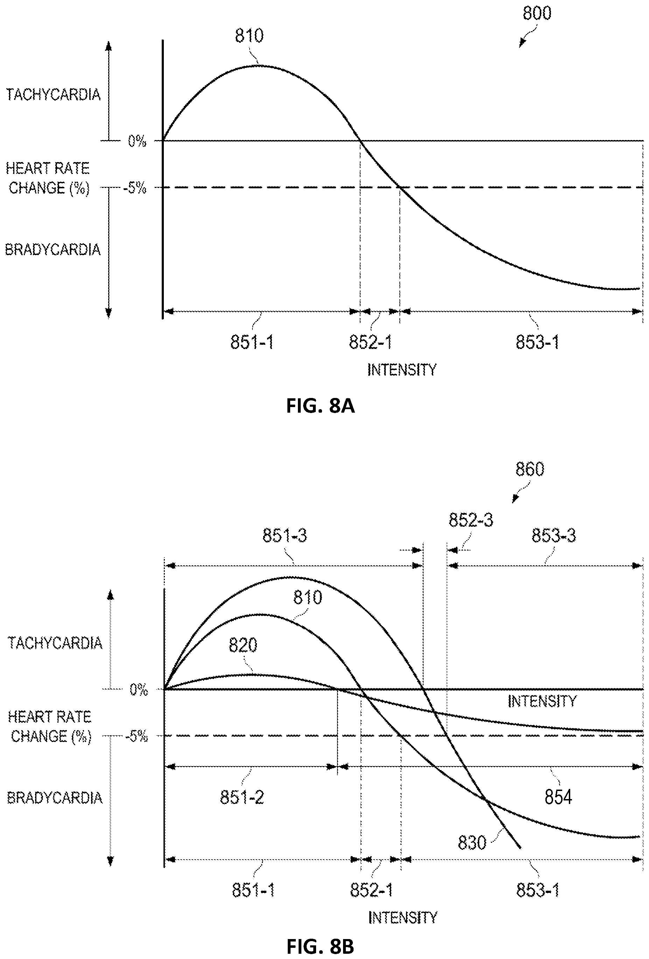

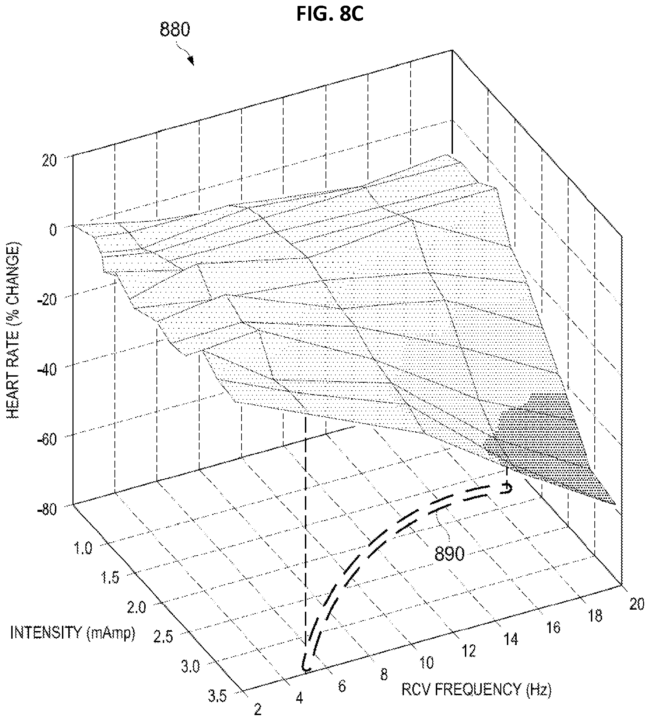

FIG. 5 is a graph 70 showing, by way of example, the relationship between the targeted therapeutic efficacy 73 and the extent of potential side effects 74 resulting from use of the implantable neurostimulator 12 of FIG. 1, after the patient has completed the titration process. The graph in FIG. 5 provides an illustration of the failure of increased stimulation intensity to provide additional therapeutic benefit, once the stimulation parameters have reached the neural fulcrum zone, as will be described in greater detail below with respect to FIG. 8. As shown in FIG. 5, the x-axis represents the duty cycle 71. The duty cycle is determined by dividing the stimulation ON time by the sum of the ON and OFF times of the neurostimulator 12 during a single ON-OFF cycle. However, the stimulation time may also include ramp-up time and ramp-down time, where the stimulation frequency exceeds a minimum threshold (as further described infra with reference to FIG. 7). When including the ramp-up and ramp-down times, the total duty cycle may be calculated as the ON time plus the ramp-up and ramp-down times divided by the OFF time, ON time, and ramp-up and ramp-down times, and may be, e.g., between 15% and 30%, and more specifically approximately 23%. The y-axis represents physiological response 72 to VNS therapy. The physiological response 72 can be expressed quantitatively for a given duty cycle 71 as a function of the targeted therapeutic efficacy 73 and the extent of potential side effects 74, as described infra. The maximum level of physiological response 72 ("max") signifies the highest point of targeted therapeutic efficacy 73 or potential side effects 74.

Targeted therapeutic efficacy 73 and the extent of potential side effects 74 can be expressed as functions of duty cycle 71 and physiological response 72. The targeted therapeutic efficacy 73 represents the intended effectiveness of VNS in provoking a beneficial physiological response for a given duty cycle and can be quantified by assigning values to the various acute and chronic factors that contribute to the physiological response 72 of the patient 10 due to the delivery of therapeutic VNS. Acute factors that contribute to the targeted therapeutic efficacy 73 include beneficial changes in heart rate variability and increased coronary flow, reduction in cardiac workload through vasodilation, and improvement in left ventricular relaxation. Chronic factors that contribute to the targeted therapeutic efficacy 73 include improved cardiovascular regulatory function, as well as decreased negative cytokine production, increased baroreflex sensitivity, increased respiratory gas exchange efficiency, favorable gene expression, renin-angiotensin-aldosterone system down-regulation, anti-arrhythmic, anti-apoptotic, and ectopy-reducing anti-inflammatory effects. These contributing factors can be combined in any manner to express the relative level of targeted therapeutic efficacy 73, including weighting particular effects more heavily than others or applying statistical or numeric functions based directly on or derived from observed physiological changes. Empirically, targeted therapeutic efficacy 73 steeply increases beginning at around a 5% duty cycle, and levels off in a plateau near the maximum level of physiological response at around a 30% duty cycle. Thereafter, targeted therapeutic efficacy 73 begins decreasing at around a 50% duty cycle and continues in a plateau near a 25% physiological response through the maximum 100% duty cycle.

The intersection 75 of the targeted therapeutic efficacy 73 and the extent of potential side effects 74 represents one optimal duty cycle range for VNS. FIG. 6 is a graph 80 showing, by way of example, the optimal duty cycle range 83 based on the intersection 75 depicted in FIG. 5. The x-axis represents the duty cycle 81 as a percentage of stimulation time over stimulation time plus inhibition time. The y-axis represents therapeutic points 82 reached in operating the neurostimulator 12 at a given duty cycle 81. The optimal duty cycle range 83 is a function 84 of the intersection 75 of the targeted therapeutic efficacy 73 and the extent of potential side effects 74. The therapeutic operating points 82 can be expressed quantitatively for a given duty cycle 81 as a function of the values of the targeted therapeutic efficacy 73 and the extent of potential side effects 74 at the given duty cycle shown in the graph 70 of FIG. 5. The optimal therapeutic operating point 85 ("max") signifies a tradeoff that occurs at the point of highest targeted therapeutic efficacy 73 in light of lowest potential side effects 74 and that point will typically be found within the range of a 5% to 30% duty cycle 81. Other expressions of duty cycles and related factors are possible.

Therapeutically and in the absence of patient physiology of possible medical concern, such as cardiac arrhythmias, VNS is delivered in a low level maintenance dose that uses alternating cycles of stimuli application (ON) and stimuli inhibition (OFF) that are tuned to activate both afferent and efferent pathways. Stimulation results in parasympathetic activation and sympathetic inhibition, both through centrally-mediated pathways and through efferent activation of preganglionic neurons and local circuit neurons. FIG. 7 is a timing diagram showing, by way of example, a stimulation cycle and an inhibition cycle of VNS 90, as provided by implantable neurostimulator 12 of FIG. 1. The stimulation parameters enable the electrical stimulation pulse output by the neurostimulator 12 to be varied by both amplitude (output current 96) and duration (pulse width 94). The number of output pulses delivered per second determines the signal frequency 93. A pulse width in the range of 100 to 250 μsec delivers between 0.02 mA and 50 mA of output current at a signal frequency of about 10 Hz, although other therapeutic values could be used as appropriate. In general, the stimulation signal delivered to the patient may be defined by a stimulation parameter set comprising at least an amplitude, a frequency, a pulse width, and a duty cycle.

The stimulation time may be considered the time period during which the neurostimulator 12 is ON and delivering pulses of stimulation, and the OFF time is considered the time period occurring in-between stimulation times during which the neurostimulator 12 is OFF and inhibited from delivering stimulation or configured to deliver a negligible or ineffective stimulation.

As shown in FIG. 7, the neurostimulator 12 may implement a stimulation time 91 comprising an ON time 92, a ramp-up time 97 and a ramp-down time 98 that respectively precede and follow the ON time 92. The ON time 92 is considered to be a time during which the neurostimulator 12 is ON and delivering pulses of stimulation at the full output current 96. The OFF time 95 is considered to comprise the ramp-up time 97 and ramp-down time 98, which are used when the stimulation frequency is at least 10 Hz, although other minimum thresholds could be used, and both ramp-up and ramp-down times 97, 98 last two seconds, although other time periods could also be used. The ramp-up time 97 and ramp-down time 98 allow the strength of the output current of each output pulse to be gradually increased and decreased, thereby avoiding deleterious reflex behavior due to sudden delivery or inhibition of stimulation at a programmed intensity corresponding to the full output current 96.

Therapeutic vagus neural stimulation has been shown to provide cardioprotective effects. Although delivered in a maintenance dose having an intensity that is insufficient to elicit undesirable side effects, such as cardiac arrhythmias, ataxia, coughing, hoarseness, throat irritation, voice alteration, or dyspnea, therapeutic VNS can nevertheless potentially ameliorate pathological tachyarrhythmias in some patients. Although VNS has been shown to decrease defibrillation threshold, VNS has not been shown to terminate VF in the absence of defibrillation. VNS prolongs ventricular action potential duration, so may be effective in terminating VT. In addition, the effect of VNS on the AV node may be beneficial in patients with AF by slowing conduction to the ventricles and controlling ventricular rate.

**Neural Fulcrum Zone**

As described above, autonomic regulation therapy results in simultaneous creation of action potentials that simultaneously propagate away from the stimulation site in afferent and efferent directions within axons comprising the cervical vagus nerve complex. Upon stimulation of the cervical vagus nerve, action potentials propagate away from the stimulation site in two directions, efferently toward the heart and afferently toward the brain. Different parameter settings for the neurostimulator 12 may be adjusted to deliver varying stimulation intensities to the patient. The various stimulation parameter settings for current VNS devices include output current amplitude, signal frequency, pulse width, signal ON time, and signal OFF time.

When delivering neurostimulation therapies to patients, it is generally desirable to avoid stimulation intensities that result in either excessive tachycardia or excessive bradycardia. However, researchers have typically utilized the patient's heart rate changes as a functional response indicator or surrogate for effective recruitment of nerve fibers and engagement of the autonomic nervous system elements responsible for regulation of heart rate, which may be indicative of therapeutic levels of VNS. Some researchers have proposed that heart rate reduction caused by VNS stimulation is itself beneficial to the patient.

According to a method which is not part of the claimed invention, a neural fulcrum zone is identified, and neurostimulation therapy is delivered within the neural fulcrum zone. This neural fulcrum zone corresponds to a combination of stimulation parameters at which autonomic engagement is achieved but for which a functional response determined by heart rate change is nullified due to the competing effects of afferently and efferently-transmitted action potentials. In this way, the tachycardia-inducing stimulation effects are offset by the bradycardia-inducing effects, thereby minimizing side effects such as significant heart rate changes while providing a therapeutic level of stimulation. One method of identifying the neural fulcrum zone is by delivering a plurality of stimulation signals at a fixed frequency but with one or more other parameter settings changed so as to gradually increase the intensity of the stimulation.

FIGS. 8A-8C provide illustrative charts reflecting the location of the neural fulcrum zone. FIG. 8A is a chart 800 illustrating a heart rate response in response to such a gradually increased intensity at a first frequency. In this chart 800, the x-axis represents the intensity level of the stimulation signal, and the y-axis represents the observed heart rate change from the patient's baseline basal heart rate observed when no stimulation is delivered. In this example, the stimulation intensity is increased by increasing the output current amplitude.

A first set 810 of stimulation signals is delivered at a first frequency (e.g., 10 Hz). Initially, as the intensity (e.g., output current amplitude) is increased, a tachycardia zone 851-1 is observed, during which period, the patient experiences a mild tachycardia. As the intensity continues to be increased for subsequent stimulation signals, the patient's heart rate response begins to decrease and eventually enters a bradycardia zone 853-1, in which a bradycardia response is observed in response to the stimulation signals. As described above, the neural fulcrum zone is a range of stimulation parameters at which the functional effects from afferent activation are balanced with or nullified by the functional effects from efferent activation to avoid extreme heart rate changes while providing therapeutic levels of stimulation. The neural fulcrum zone 852-1 can be located by identifying the zone in which the patient's response to stimulation produces either no heart rate change or a mildly decreased heart rate change (e.g., <5% decrease, or a target number of beats per minute). As the intensity of stimulation is further increased at the fixed first frequency, the patient enters an undesirable bradycardia zone 853-1. In these examples, the patient's heart rate response is used as an indicator of autonomic engagement. In other examples, other physiological responses may be used to indicate the zone of autonomic engagement at which the propagation of efferent and afferent action potentials are balanced to identify the neural fulcrum zone.

FIG. 8B is a chart 860 illustrating a heart rate response in response to such a gradually increased intensity at two additional frequencies. In this chart 860, the x-axis and y-axis represent the intensity level of the stimulation signal and the observed heart rate change, respectively, as in FIG. 8A, and the first set 810 of stimulation signals from FIG. 8A is also shown.

A second set 820 of stimulation signals is delivered at a second frequency lower than the first frequency (e.g., 5 Hz). Initially, as the intensity (e.g., output current amplitude) is increased, a tachycardia zone 851-2 is observed, during which period, the patient experiences a mild tachycardia. As the intensity continues to be increased for subsequent stimulation signals, the patient's heart rate response begins to decrease and eventually enters a mild heart rate reduction zone 854, in which a mild decrease in heart rate is observed in response to the stimulation signals. The low frequency of the stimulation signal in the second set 820 of stimulation signals limits the functional effects of nerve fiber recruitment and, as a result, the heart response remains relatively limited. Although this low frequency stimulation results in minimal heart rate reduction, and, therefore, minimal side effects, the stimulation intensity is too low to result in effective recruitment of nerve fibers and engagement of the autonomic nervous system. As a result, a therapeutic level of stimulation is not delivered.

A third set of 830 of stimulation signals is delivered at a third frequency higher than the first and second frequencies (e.g., 20 Hz). As with the first set 810 and second set 820, at lower intensities, the patient first experiences a tachycardia zone 851-3. At this higher frequency, the level of increased heart rate is undesirable. As the intensity is further increased, the heart rate decreases, similar to the decrease at the first and second frequencies but at a much higher rate. The patient first enters the neural fulcrum zone 852-3 and then the undesirable bradycardia zone 853-3. Because the slope of the curve for the third set 830 is much steeper than the first set 810, the region in which the patient's heart rate response is between 0% and -5% (e.g., the neural fulcrum zone 852-3) is much narrower than the neural fulcrum zone 852-1 for the first set 810. Accordingly, when testing different operational parameter settings for a patient by increasing the output current amplitude by incremental steps, it can be more difficult to locate a programmable output current amplitude that falls within the neural fulcrum zone 852-3. When the slope of the heart rate response curve is high, the resulting heart rate may overshoot the neural fulcrum zone and create a situation in which the functional response transitions from the tachycardia zone 851-3 to the undesirable bradycardia zone 853-3 in a single step. At that point, the clinician would need to reduce the amplitude by a smaller increment or reduce the stimulation frequency in order to produce the desired heart rate response for the neural fulcrum zone 852-3.

FIG. 8C is a chart 880 illustrating mean heart rate response surfaces in conscious, normal dogs during 14 second periods of right cervical vagus VNS stimulation ON-time. The heart rate responses shown in z-axis represent the percentage heart rate change from the baseline heart rate at various sets of VNS parameters with the pulse width set at 250 μsec, the pulse amplitude ranging from 0 mA to 3.5 mA (provided by the x-axis), and the pulse frequency ranging from 2 Hz to 20 Hz (provided by the y-axis). Curve 890 roughly represents the range of stimulation amplitude and frequency parameters at which a null response (i.e., 0% heart rate change from baseline) is produced. This null response curve 890 is characterized by the opposition of functional responses (e.g., tachycardia and bradycardia) arising from afferent and efferent activation.

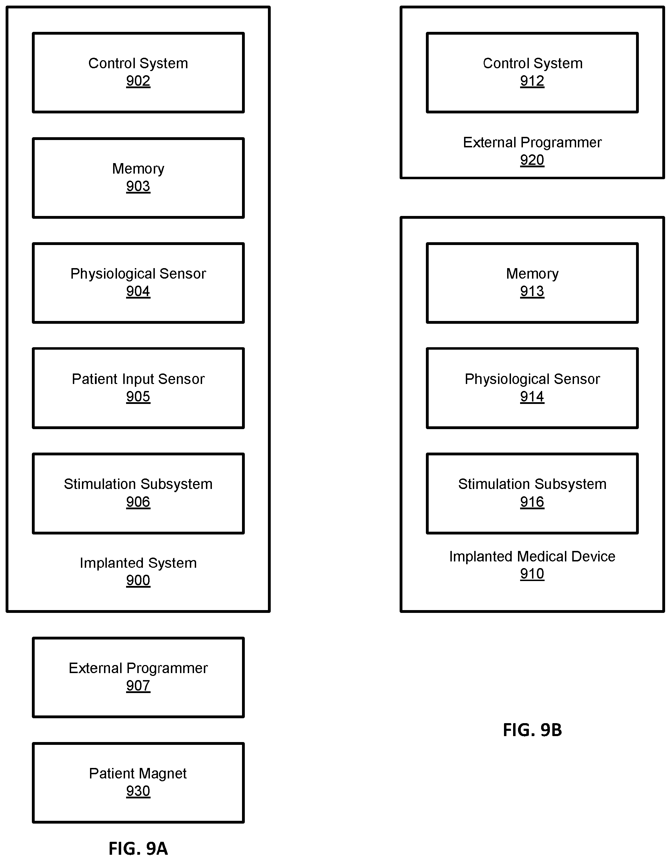

FIG. 9A is a simplified block diagram of an implanted neurostimulation system 900 in accordance with embodiments of the present invention. The implanted neurostimulation system 900 comprises a control system 902 comprising a processor programmed to operate the system 910, a memory 903, an optional physiological sensor 904, and a stimulation subsystem 906. The physiological sensor 904 may be configured to monitor any of a variety of patient physiological signals and the stimulation subsystem 906 may be configured to deliver a stimulation signal to the patient. In one example, the physiological sensor 904 comprises an ECG sensor for monitoring heart rate and the stimulation subsystem 906 comprises a neurostimulator 12 programmed to deliver ON-OFF cycles of stimulation to the patient's vagus nerve.

The control system 902 is programmed to activate the neurostimulator 12 to deliver varying stimulation intensities to the patient and to monitor the physiological signals in response to those stimulation signals.

The external programmer 907 shown in FIG. 9A may be utilized by a clinician or by the patient for communicating with the implanted system 910 to adjust parameters, activate therapy, retrieve data collected by the system 910 or provide other input to the system 910. In some embodiments, the external programmer 907 may be configured to program the implanted system 910 with a prescribed time or window of time during which titration sessions may be initiated. This can be used to prevent a titration session from occurring at night when the patient's sleep is likely to be disturbed by the increase in stimulation intensity and resulting side effects.

Patient inputs to the implanted system 910 may be provided in a variety of ways. The implanted system 910 may include a patient input sensor 905. As described above, a patient magnet 930 may be used to provide external input to the system 910. When the patient magnet 930 is placed on the patient's chest in close proximity to the implanted system 910, the patient input sensor 905 will detect the presence of the magnetic field generated by the patient magnet 930 and provide a control input to the control system 902. The system 910 may be programmed to receive patient inputs to set the time of day during which titration sessions are to be initiated.

In other embodiments, the patient input sensor 905 may comprise a motion sensor, such as an accelerometer, which is configured to detect intentional tapping on the surface of the patient's chest as a form or communication with the system. The patient may use finger taps in one or more predetermined patterns to provide certain control inputs to the implanted system 910. For example, the motion sensor may detect three rapid taps to the patient's chest in the general proximity or more specifically, anterior (ventral) to the implanted pulse generator and the resultant pressure waves propagating in the body's interior including the volume of tissue incorporating the implanted pulse generator may trigger an operation on the implanted system 910 (e.g., to initiate a titration session). In other embodiments, the patient input sensor 905 may comprise an acoustic transducer or other sensor configured to detect acoustic signals. The system 910 may be programmed to interpret the detection of certain sounds as patient inputs. For example, the patient may utilize an electronic device, such as a smartphone or other portable audio device, to generate one or more predetermined sequences of tones. The system 910 may be programmed to interpret each of these sequences of tones as a different patient input.

The delivery of the stimulation signal and the monitoring of the patient's physiological response (e.g.,

heart rate or other statistical derivative of the measured heart period (beat-to-beat interval)) may be advantageously implemented using control system in communication with both the stimulation subsystem 906 and one or more physiological sensors 904, such as by incorporating all of these components into a single implantable device. In accordance with other embodiments, the control system may be implemented in a separate implanted device or in an external programmer 920 or other external device, as shown in FIG. 9B. The external programmer 920 in FIG. 9B may be utilized by a clinician or by the patient for adjusting stimulation parameters. The external programmer 920 includes a control system 912 and is in wireless communication with the implanted medical device 910, which includes the stimulation subsystem 916. In the illustrated embodiment, the physiological sensor 914 is incorporated into the implanted medical device 910, but in other embodiments, the sensor 914 may be incorporated into a separate implanted device, may be provided externally and in communication with the external programmer 920, or may be provided as part of the external programmer 920. The implanted medical device 910 may further include a memory 913 that may store information in preparation for a transmission to the external programmer 920.

**Intrinsic Heart Rate Recovery**

Heart Rate Recovery (HRR) represents the spontaneous gradual decrease in heart rate (HR) following a spontaneous increase in heart rate that often occurs in connection with emotional changes or cessation of physical exertion (activities of daily living, including exercise) and is an index of parasympathetic activity. Physical exertion is associated with increased sympathetic and decreased parasympathetic activity. After a period of physical exertion, there is a heart rate recovery period during which the patient's HR gradually returns to a new level, typically its pre-exercise level. Patients with CHF have autonomic nervous system dysfunction, including abnormally prolonged HRR.

HRR is typically assessed in a patient upon completion of a specified and controlled exercise protocol, which can provoke a controlled autonomic response. However, the health condition of CHF patients may be so impaired that they lack the physical ability to perform the specified exercise protocol, and therefore incapable of producing the controlled response. As a result, conventional methods of assessing HRR using controlled protocols are not possible.

Autonomic regulation therapy (ART) via vagus nerve stimulation (VNS) has been shown to improve autonomic balance and ventricular function in HF patients. In accordance with embodiments of the present invention, automated analysis of a patient's Intrinsic Heart Rate Recovery (IHRR) can be used as a valuable tool for assessing a CHF patient's autonomic nervous system function and improving the treatment of CHF using VNS.

This new approach enables the assessment of HRR using easily acquired heart rate data from periods of elevated heart rate that occur naturally during a patient's activities of daily living. This approach does not require the controlled exercise protocol to provoke a controlled autonomic response that may otherwise exclude some patients (although a controlled exercise protocol may also be applied, if desired).

IHRR presents a distribution of a relaxation parameter associated with heart rate change and patient's autonomic condition. It is noted from actual data that this semi-stationary relaxation parameter changes with observed change in heart rate as well as a patient's autonomic status. This is a possible behavior of a system with some degree of nonlinearity where the amplitude of the perturbation affects the relaxation time constant. A method for performing the measurements is described in greater detail below.

IHRR may be assessed and quantified in a variety of ways. One option is to model identified heart rate deceleration as a mono-exponential decay at multiple time points before, during and after recovery and to estimate the time constant ($T_R$) of HR decay. The equation of the trend line for this decay is:

$$HR(t) = a_0 + b \cdot e^{(-1/T_R) \cdot t}$$

In this equation, $a_0$ is the asymptotic value of the HR, b is the decremented value below the peak HR, $T_R$ is the time constant, and $e$ is a mathematical constant. The time constant ($T_R$) reflects HRR such that a higher time constant reflects a faster HRR. Other polynomial equations or even non-parametric models can also be used to model the effective deceleration of HR.

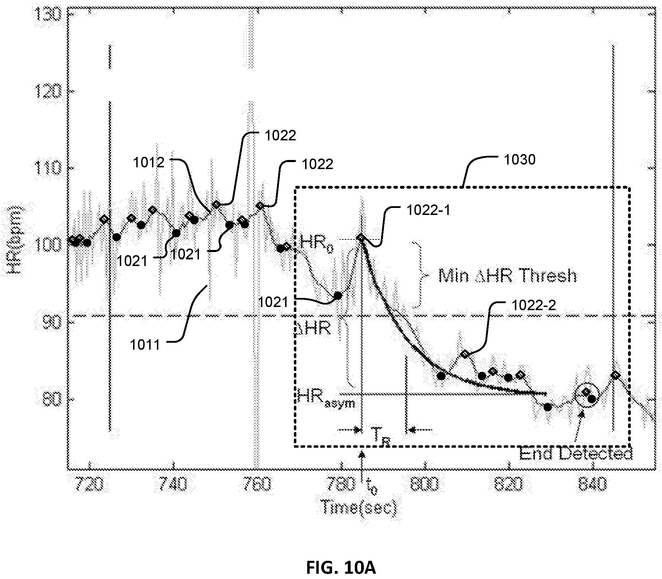

FIGS. 10A-10B are charts illustrating a method of extracting an Intrinsic Heart Rate Recovery (IHRR) parameter. In one study, the heart rate for HF patients was measured for 24-hour periods using externally worn Holter monitors to measure the patients' ECG signals. This data was used to establish a criteria for identifying certain episodes of heart rate increase as intrinsic heart rate increase events of interest.

In FIG. 10A, the x-axis represents a portion of the 24-hour monitoring period (although other recording durations may be acceptable), and the y-axis represents the patient's heart rate in beats per minute (bpm). The first line 1011 shows the instantaneous heart rate measurements by the Holter monitor. According to the invention, in order to remove isolated heart rate changes and other random variability, the heart rate is smoothed by calculating a moving median followed by a moving mean of a subset of beats (e.g., 3-5 beats or less than 10 beats). This smoothed heart rate is shown by the second line 1012. Within this smoothed heart rate 1012, paired local minima points 1021 and local maxima points 1022 are identified. According to the invention, these pairs of minima points 1021 and maxima points 1022 are analyzed using software to identify candidate events in the heart rates.

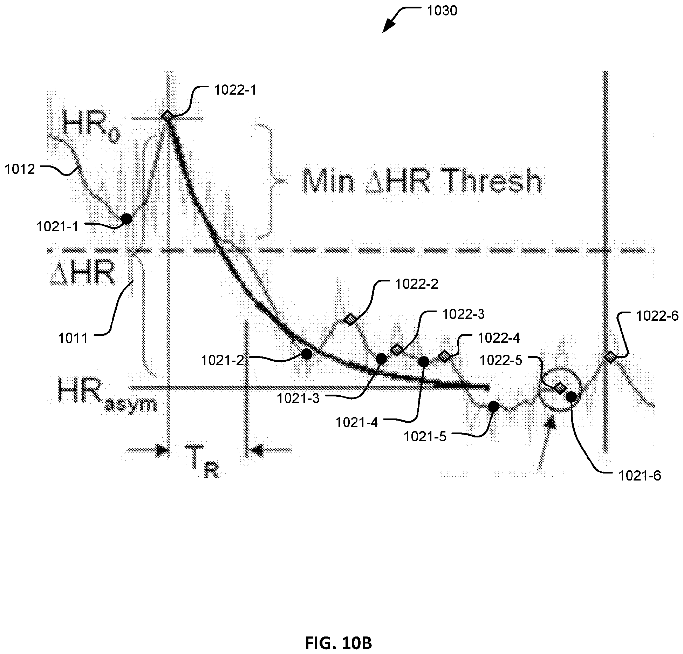

Any of a variety of algorithms may be used to identify the candidate events, but in the illustrated example, the sequence of paired maxima points 1022 and minima points 1021 are analyzed to identify a point where there is a decrease in the smoothed heart rate of at least a specified minimum $\Delta HR$ threshold number of beats per minute (bpm) (five bpm in this example) between the initial $HR_0$ and the final $HR_{asym}$. FIG. 10B is an enlarged view of an IHRR region of interest 1030 shown in FIG. 10A. In the example shown in FIG. 10B, there is a drop of approximately 13 bpm represented by the set of points comprising the initial maxima ($HR_0$) point 1022-1, subsequent maxima points 1022-2 through 1022-5, and subsequent minima points 1021-2 through 1021-6. Once this criteria is met, the initial maxima point 1022-1 is established as the beginning of the candidate region of interest 1030.

Once the initial maximum point 1022-1 is identified, the subsequent maxima points 1022 and minima points 1021 are analyzed to determine whether two criteria are satisfied: first, that each maxima point 1022 is less than the immediately preceding maxima point 1022, and second, that each minima point 1021 is less than the initial maxima point 1022 and the immediately preceding minima point 1021. Here, the first maxima point 1022-1 represents the beginning of the candidate event. It can be seen that each subsequent maxima point 1022-2 through 1022-5 is less than the immediately preceding maxima point, and each subsequent minima point 1021-2 through 1021-5 is less than the initial maxima point 1022-1 and the immediately preceding minima point, which represents a gradually decreasing heart rate indicative of the patient's heart rate recovery. Finally, the heart rate reaches minima point 1021-6, which is greater than the immediately preceding minima point 1021-5. This fails the second criteria and therefore establishes the end of the region of interest 1030. The maxima points 1022-1 through 1022-5 and the minima points 1021-2 through 1021-6 are then identified as forming the candidate region of interest.

A less strict criterion for determining the end of the region of interest 1030 can be used to identify a predefined number of maxima-minima pairs as described above within a predetermined time window. However, the pairs do not have to occur in a consecutive order. In such a case, the end of the region of interest is marked following the identification of the last maxima-minima pair. A variation on the above method is to reverse the above steps, e.g., first test all smoothed maxima one-by-one applying the previously described monotone decreasing requirements to the minima and maxima then apply the minimum $\Delta HR$ threshold to the surviving sequences as described above.

In order to provide a more meaningful analysis, it may be desirable to extract only those candidate events which include a minimum number of maxima-minima pairs within the region of interest. In the illustrated example, only those candidate events which include at least three maxima-minima pairs are identified as an Intrinsic Heart Rate Recovery (IHRR) event of interest for further analysis. The candidate events having less than three maxima-minima pairs would be considered to provide too small of a sample size to provide meaningful insight into the patient's heart rate recovery. When the candidate event includes only a small number of pairs, there may be many other explanations for the change in heart rate unrelated to the sympathetic-parasympathetic interaction occurring during HRR. By requiring a minimum number of maxima-minima pairs in the candidate event, the event is more likely to capture events that are indicative of how the patient's sympathetic and parasympathetic systems are interacting.

In some cases, it may also be desirable for the candidate event to occur over no more than a maximum period of time. For example, it may be desirable to identify only those candidate events in which the initial maxima point 1022-1 and final minima point 1021-6 occur over a period of less than 1-2 minutes.

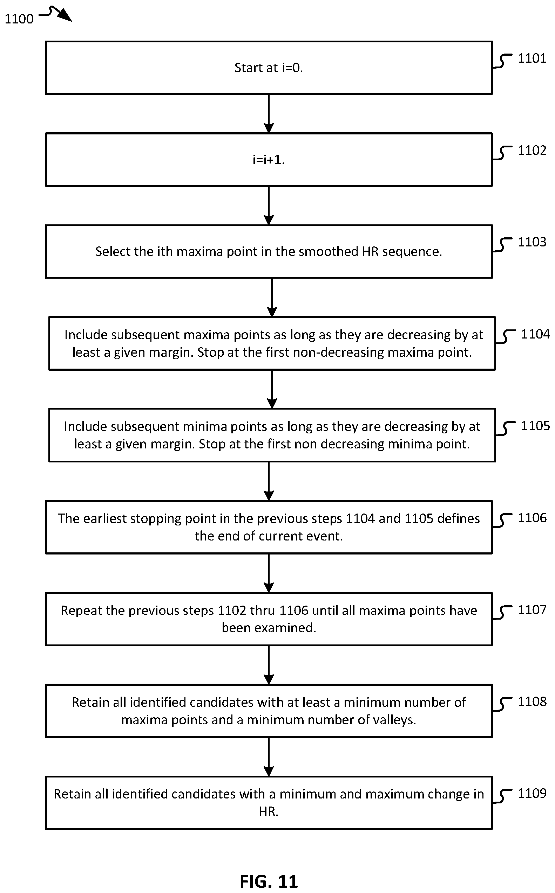

FIG. 11 is a flowchart illustrating one method 1100 of identifying IHRR events from smoothed heart rate data. Step 1101 begins with i=0, where i represents a candidate local maxima point. Initially, all smoothed local maxima may be considered initiating points of potential candidate events. In step 1102, i is incremented by one. In step 1103, the ith maxima point in the smoothed HR sequence is selected. In step 1104, subsequent maxima points are included as long as they decrease by at least a given margin. Stop at the first non-decreasing maxima point. In step 1105, include subsequent minima points as long as they are decreasing by at least a given margin. Stop at the first non-decreasing minima point. In step 1106, the earliest stopping point in the previous steps 1104 and 1105 defines the end of current candidate event. In step 1107, repeat the previous steps 1102-1106 until all candidate maxima points have been examined. In step 1108, retain all identified candidates with at least a minimum number of maxima points and a minimum number of minima points. In step 1109, retain all identified candidates with greater than a minimum specified and less than a maximum specified change in HR. As mentioned above, the steps of FIG. 11 may executed before considering the minimum HR change requirement as a variation of the event identification process.

After all of the IHRR events in the data set are identified, a first order exponential is taken of each identified IHRR event in the heart rate data set to produce a modeled time constant ($T_R$) and a corresponding modeled change in heart rate ($\Delta HR$) as in FIG. 10B from data starting with point 1022-1 and including subsequent maxima points 1022-2 through 1022-5 and subsequent minima points 1021-2 through 1021-5. Variations of the first order exponential modeling may be derived using subsets of the minima and maxima to model the mono-exponential decay such as, e.g.: (i) all of the maxima in the set including the first maximum, (ii) all of the minima including the first minimum or (iii) all of the minima and maxima including the first maximum in the set. The calculated time constant for the IHRR event is identified as the relaxation parameter $T_R$ for that IHRR event. The IHRR event provides an indication of how quickly the patient's heart rate returns to baseline after a period of exertion. HF patients typically take much longer to return to baseline than healthy patients. The relaxation parameter is a measure of the speed with which the patient's heart rate returns to baseline during each IHRR event, and each relaxation parameter is associated with a corresponding magnitude of HR change.

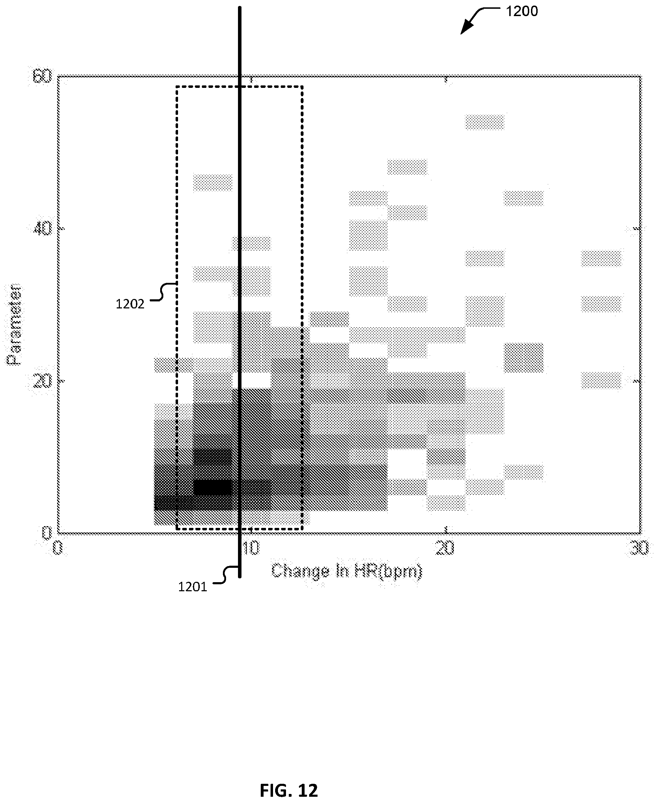

FIG. 12 is a histogram 1200 of the relaxation parameters plotted against the corresponding change in heart rate for a single patient over a certain period of time. In the illustrated example, this data corresponds to the IHRR events identified over a twenty-four hour period of time. Shorter or longer periods of time may be analyzed.

A rapid HRR rate is indicative of physical fitness, as a healthy patient's heart is capable returning to baseline more quickly than a HF patient's heart. Accordingly, the relaxation parameter time constant in the y-axis for a healthy patient would be less elevated across all heart rate changes in the x-axis. As shown in FIG. 12, the relaxation parameter time constant for an HF patient would be somewhat elevated across the chart.

Next, the mean relaxation parameter for each HR change is calculated. In order to include sufficient data points for each HR change level, the mean is taken of the relaxation parameters in a band around each HR change level. As shown in FIG. 12, the HR change level of 9 bpm is identified by line 1201. A banded region 1202 around the 9 bpm level is drawn, and the mean is taken of all of the relaxation parameters within that banded region 1202. The width of the banded region 1202 may vary, depending on the size of the data set, but in the illustrated example, the banded region 1202 represented +/- 4 bpm around the 9 bpm level, i.e., the banded region 1202 extends from 5 bpm to 13 bpm.

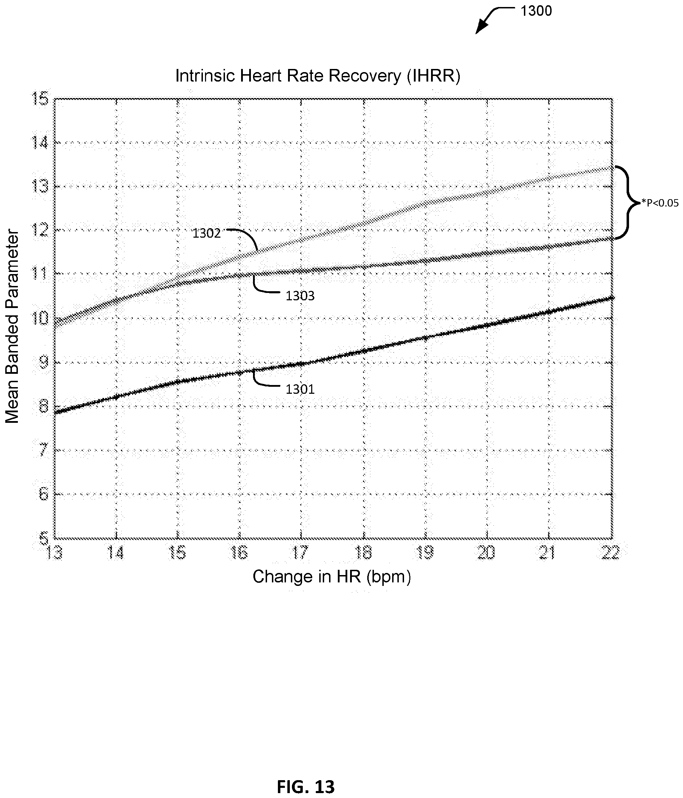

The mean banded relaxation parameter is then calculated for each change in HR level in the histogram 1200 and then plotted on the line chart 1300 in FIG. 13. The line chart 1300 is a line chart of the mean banded relaxation parameter (y-axis) as a function of the corresponding change in HR (x-axis) for three different data sets. The first line 1301 represents the mean banded relaxation parameter for a cohort of healthy normal patients. The second line 1302 represents the mean banded relaxation parameters for a cohort of heart failure patients, where the heart rate data was collected during a screening period prior to initiating the autonomic regulation therapy via vagus nerve stimulation. The third line 1303 represents the mean banded relaxation parameter for that same cohort of heart failure patients, where the heart rate data was collected during at a follow-up evaluation six months after the autonomic regulation therapy was initiated. It is to be noted that the banded relaxation parameter may be calculated in a variety of ways, including, for example, as a median banded relaxation parameter.

As can be seen in FIG. 13, the first line 1301 remains lower across all HR change levels, which reflects the fact that a healthy patient presents the lowest index of heart rate recovery. The decrease from second line 1302 to third line 1303 at higher HR change levels reflects that the autonomic regulation therapy via VNS was, on average, successful at improving the HRR for the cohort of HF patients. This suggests that chronic autonomic regulation therapy in patients with symptomatic HF may result in a partial normalization of parasympathetic activity and autonomic nervous system regulation of heart rate, as assessed by intrinsic HRR. This may offer a potential clinical benefit to patients not currently provided by existing heart failure therapies. In addition, analysis of the IHRR in a patient undergoing ART therapy may be used to determine the efficacy of the therapy, as reflected by improvement in the patient's IHRR. If the patient's IHRR does not improve between the initial screening and the follow-up evaluation, the clinician may conclude that the patient is not responding to the therapy and may adjust therapy parameters to try to achieve some improvement, or discontinue therapy altogether if it is concluded that the patient is a non-responder to the therapy. Observed changes in the patient's IHRR may also be used to optimize therapy by enabling the clinician to adjust parameters until the maximum IHRR improvement is achieved.

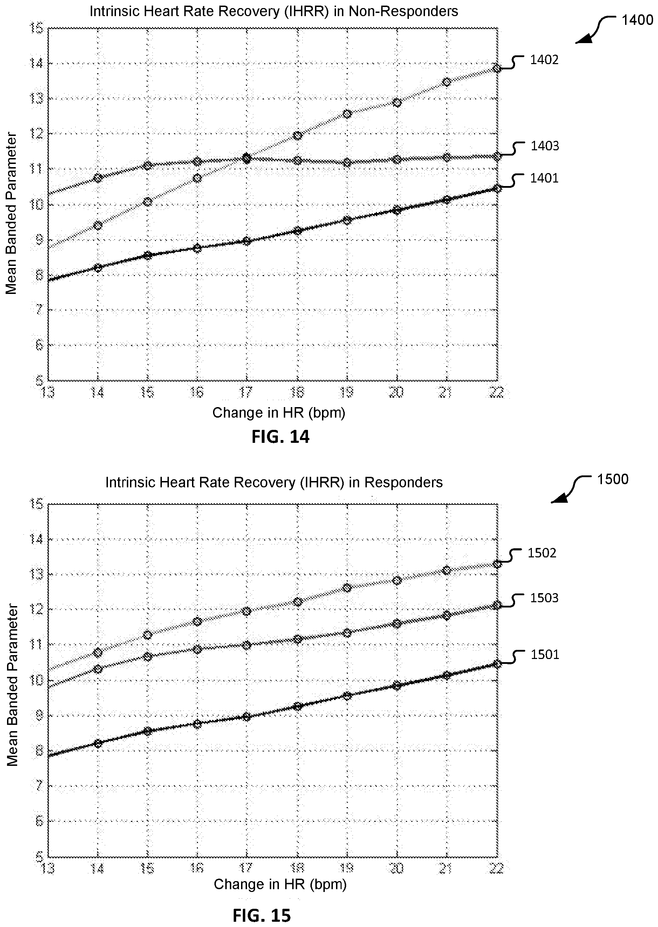

The patient's IHHR may be used to improve therapy in additional ways. FIG. 14 illustrates the mean banded relaxation parameters as a function of the corresponding change in HR for a subgroup of patients who were deemed to be non-responders to the ART therapy, and FIG. 15 illustrates the mean banded relaxation parameters as a function of the corresponding change in HR for patients who were deemed to be responders to the ART therapy.

Left ventricular ejection fraction is a measurement of the amount of blood pumped out of the left ventricle with each heartbeat. In a healthy patient, the ejection fraction is typically about 55% to 70%. In a HF patient, the ejection fraction may be about 20% to 40%, or even less. In the cohort of HF patients whose data is depicted in FIG. 13, the left ventricular ejection fraction was measured at the six month follow-up evaluation point after initiation of ART therapy, and the patients who had a decreased ejection fraction as a result of therapy were categorized as non-responders, and the patients who had an ejection fraction change of 0% or greater were categorized as responders. The IHRR data for the non-responders and the responders are shown in charts 1400 and 1500, respectively.

In chart 1400 shown in FIG. 14, the first line 1401 represents the mean banded relaxation parameter for a healthy patient, the same as the first line 1301 in FIG. 13. The second line 1402 represents the mean banded relaxation parameters for a cohort of heart failure patients deemed to be non-responders based on the ejection fraction measurements described above, where the heart rate data was collected during a screening period prior to initiating the autonomic regulation therapy. The third line 1403 represents the mean banded relaxation parameter for that cohort of non-responders, where the heart rate data was collected during at a follow-up evaluation six months after the autonomic regulation therapy was initiated. FIG. 14 shows that in this non-responder group, there was an improvement in HRR over a subset of HR change levels (e.g., greater than 17 bpm), and there was a deterioration in HRR over another subset of HR change levels (e.g., less than 17 bpm).

In chart 1500 shown in FIG. 15, the first line 1501 represents the mean banded relaxation parameter for a group of healthy patients, the same as the first line 1301 in FIG. 13 and the first line 1401 in FIG. 14. The second line 1502 represents the mean banded relaxation parameters for a cohort of heart failure patients deemed to be responders based on the ejection fraction measurements described above, where the heart rate data was collected during a screening period prior to initiating the autonomic regulation therapy. The third line 1503 represents the mean banded relaxation parameter for that cohort of responders, where the heart rate data was collected during at a follow-up evaluation six months after the autonomic regulation therapy was initiated. FIG. 15 shows that in this responder group, there was an improvement in HRR across all HR change levels, which is consistent with the improvement measured in the patients' ejection fraction.

The results shown in FIG. 14 for the non-responder group may be used to improve therapy in a variety of ways. Notably, in the non-responder chart 1400, the difference in the mean banded relaxation parameter between the screening period data set and the healthy patient data set is smaller than the difference in the mean banded relaxation parameter between the screening period data set and the healthy patient data set in the responder chart 1500 at lower HR change levels. For example, in FIG. 14, at 13 bpm, the relaxation parameter difference between line 1401 and line 1402 for non-responders is approximately 1, whereas in FIG. 15, at 13 bpm, the relaxation parameter difference between line 1501 and line 1502 for responders is approximately 2.3. However, after ART therapy is initiated, for the non-responder cohort, the relaxation parameter difference increases from approximately 1 to approximately 1.3, whereas the relaxation parameter difference in the responder cohort decreases from approximately 2.3 to approximately 2.0. At higher HR change levels, chart 1400 shows that the difference in the relaxation parameter between the non-responder screening period data set (line 1402) and the healthy patient data set (line 1401) is much greater than the difference between lines 1502 and 1501 for responders in chart 1500. However, the difference in the relaxation parameter between the non-responder six month follow-up data set (line 1403) and the healthy patient data set (line 1401) is much smaller than the difference between lines 1502 and 1501.

It may be theorized that the responder cohort reflects a patient population that is more pathological than the non-responder cohort, and therefore in need of higher doses of stimulation therapy in order to increase the patients' parasympathetic drive. In contrast, the non-responder cohort may not be as pathological as the responder cohort, and therefore these non-responders' autonomic systems may be in less need of therapy than the responder cohort. The HRR for these non-responders is already close to normal levels at lower HR change levels without therapy, so the ART therapy may in fact be overdriving the patients' parasympathetic systems, resulting in the decreased IHRR after initiation of therapy.

Based on this observation, it may be desirable to incorporate a heart rate sensor or other activity level sensor into the implanted device and into a separate device (implanted or external) to be used in conjunction with the implanted neurostimulation device. The implanted device may then be programmed to provide increased levels of stimulation when the patient has experienced a large change in heart rate, and decreased or suspended stimulation when the patient is experiencing only small changes in heart rate. In other words, in accordance with some embodiments of the present invention, the stimulation parameters may be a function of the activity level of the patient, with low levels of stimulation (e.g., decreased stimulation frequency of 2 Hz - 5 Hz) during periods of low activity, and high levels of stimulation (e.g., increased stimulation frequency of 10 Hz) during periods of high activity.

In accordance with embodiments of the present invention, the patient's IHRR may be monitored over a period of time, either entirely during a period that stimulation therapy is being delivered or during a period of stimulation therapy delivery and during a period during which stimulation therapy is not being delivered. The IHRR may be monitored on a continuous basis, on any desired periodic schedule (e.g., every other day, one day per week, one day per month, etc.), or in response to a manual input or detection of a predetermined patient state. This IHRR may be recorded for later analysis by a clinician, so that the clinician may analyze the IHRR trend over time.

This monitoring and reporting may occur in conjunction with automated stimulation parameter adjustments described above, or may occur without any changes to the stimulation therapy. The clinician may then decide whether to make adjustments to the therapy based on the IHRR data. In some embodiments, the system may transmit an alert to the clinician that an adjustment may be desirable, based on monitored changes to the patient's IHRR.

The IHRR data may be transmitted to a clinician computer directly or via an intermediate external device, which can transmit the IHRR data over a wide area network to the clinician computer. The clinician computer may comprise a workstation in a clinician's office or a server accessible to a workstation in the clinician's office.

In accordance with various embodiments of the present invention, the monitored IHRR may be used in a variety of ways to improve the delivery of stimulation therapy to a subject. For example, the stimulator may automatically adjust one or more stimulation parameters in response to certain predetermined changes in the IHRR. The IHRR may also be analyzed by the implanted stimulation device or an external computer to recommend a change to one or more stimulation parameters. This recommendation may be delivered to the patient or health care provider, and may optionally be accompanied by the relevant historical IHRR data. A health care provider or patient may be requested to approve a suggested change to the one or more stimulation parameters before the change is made. In other embodiments, the recommended change to the stimulation parameters may transmitted to the health care provider or user, and then those changes can be made automatically unless the user or health care provider intervenes to instruct the system not to implement the change.

The claims define the matter for which protection is sought in terms of the technical features of the invention. In examples described above, the stimulation is applied to the vagus nerve. Alternatively, spinal cord stimulation (SCS) may be used in place of or in addition to vagus nerve stimulation for the above-described therapies. SCS may utilize stimulating electrodes implanted in the epidural space, an electrical pulse generator implanted in the lower abdominal area or gluteal region, and conducting wires coupling the stimulating electrodes to the generator.

## Claims

1. A neurostimulation system, comprising:

   an electrode assembly (13, 14); and
   a neurostimulator (12; 900; 910, 920) coupled to the electrode assembly, the neurostimulator comprising
   a control circuit (902; 912); and

   a memory (903; 913) operably coupled to the control circuit and comprising instructions that when executed by the control circuit, cause the control circuit to:

   deliver stimulation signals to a patient via the electrode assembly;
   increase an intensity of the stimulation signals until a tachycardia heart rate response in the patient is detected;
   continue to increase the intensity of the stimulation signals until a bradycardia heart rate response in the patient is detected;
   deliver therapeutic stimulation signals to the patient at an intensity within a neural fulcrum zone, wherein the neural fulcrum zone corresponds to a zone in which a response of the patient to stimulation is a 0% to 5% reduction from a baseline heart rate;

   calculate a moving median (1012) of a subset of heart beats over a first period of time
   identify paired maxima (1022) and minima (1021) points in the moving median; and
   identify one or more heart rate recovery events, each heart rate recovery event corresponding to a period of time during which the paired maxima and minima points satisfy a predetermined criteria.

2. The neurostimulation system of claim 1, wherein the stimulation signals comprise an output current amplitude, a pulse width, a pulse frequency, and a duty cycle.

3. The neurostimulation system of claim 2, wherein the instructions cause the control circuit to increase the intensity of the stimulation signals by increasing at least one of the output current amplitude, the pulse width, the pulse frequency, or the duty cycle of the stimulation signals.

4. The neurostimulation system of claim 1, wherein the system is configured to deliver the stimulation signals to a vagus nerve of the patient.

5. The neurostimulation system of claim 1, wherein the instructions further cause the control circuit to:

   deliver the stimulation signals to the patient via the electrode assembly at a first frequency and at a second frequency;
   for each of the first frequency and the second frequency, increase the intensity of the stimulation signals until a tachycardia heart rate response in the patient is detected;
   for each of the first frequency and the second frequency, continue to increase the intensity of the stimulation signals until a bradycardia heart rate response in the patient is detected; and
   deliver the therapeutic stimulation signals to the patient at one of the first frequency or the second frequency and at an intensity within the neural fulcrum zone at the one of the first frequency or the second frequency.

6. The neurostimulation system of claim 5, wherein the instructions cause the control circuit to increase the intensity of the stimulation signals by increasing an output current amplitude of the stimulation signals.

7.  The neurostimulation system according to claim 1, wherein the instructions cause the control circuit to extract one or more events of interest that include a minimum number of paired maxima and minima points.

8.  The neurostimulation system according to claim 1, wherein the instructions cause the control circuit to identify one or more events of interest, each event of interest corresponding to a period of time during which a predefined number of paired maxima and minima points occur following an initial maximum point.

9.  The neurostimulation system according to claim 1, wherein the instructions cause the control circuit to:

    initiate a stimulation therapy in the patient with the neurostimulator;
    monitor an activity level of the patient to identify a change in the activity level; and
    modify one or more stimulation signals in response to the change in the activity level of the patient.

10. The neurostimulation system according to claim 9, wherein the instructions cause the control circuit to monitor the activity level of the patient by monitoring a heart rate of the patient.

11. The neurostimulation system according to claim 9, wherein the instructions cause the control circuit to modify the one or more stimulation signals by decreasing a stimulation intensity during a period of low activity level and increasing the stimulation intensity during a period of high activity level.

**Patentansprüche**

1.  Ein Neurostimulationssystem, das umfasst:

    eine Elektrodenanordnung (13, 14); und
    einen Neurostimulator (12; 900; 910, 920), der mit der Elektrodenanordnung verbunden ist, wobei der Neurostimulator umfasst:

        eine Steuerschaltung (902; 912); und
        einen Speicher (903; 913), der betriebsfähig mit der Steuerschaltung verbunden ist und Anweisungen enthält, die, wenn sie von der Steuerschaltung ausgeführt werden, die Steuerschaltung veranlassen:

            Stimulationssignale über die Elektrodenanordnung an einen Patienten abzugeben;
eine Intensität der Stimulationssignale zu erhöhen, bis eine Tachykardie-Herzratenreaktion bei dem Patienten detektiert wird;
die Intensität der Stimulationssignale weiter zu erhöhen, bis eine bradykarde Herzratenreaktion bei dem Patienten detektiert wird;
therapeutische Stimulationssignalen an den Patienten mit einer Intensität innerhalb einer neuralen Fulcrumzone abzugeben, wobei die neurale Fulcrumzone einer Zone entspricht, in der eine Reaktion des Patienten auf die Stimulation eine 0%- bis 5%-ige Reduktion gegenüber einer Grundlinienherzrate ist;
eines gleitenden Median (1012) einer Untergruppe von Herzschlägen über eine erste Zeitperiode zu berechnen;
gepaarte Maxima (1022) und Minima (1021) in dem gleitenden Median zu identifizieren; und
ein oder mehrere Herzrate-Erholungsereignisse zu identifizieren, wobei jedes Herzrate-Erholungsereignis einer Zeitperiode entspricht, während der die gepaarten Maxima und Minima ein vorbestimmtes Kriterium erfüllen.

2.  Das Neurostimulationssystem nach Anspruch 1, wobei die Stimulationssignale eine Ausgangsstromamplitude, eine Pulsbreite, eine Pulsfrequenz und einen Arbeitszyklus umfassen.

3.  Das Neurostimulationssystem nach Anspruch 2, wobei die Anweisungen die Steuerschaltung veranlassen, die Intensität der Stimulationssignale durch Erhöhen zumindest von einem von der Ausgangsstromamplitude, der Pulsbreite, der Pulsfrequenz und/oder dem Arbeitszyklus der Stimulationssignale zu erhöhen.

4.  Das Neurostimulationssystem nach Anspruch 1, wobei das System dazu ausgebildet ist, die Stimulationssignale an einen Vagusnerv des Patienten abzugeben.

5.  Das Neurostimulationssystem nach Anspruch 1, wobei die Anweisungen die Steuerschaltung weiterhin veranlassen:

    die Stimulationssignale über die Elektrodenanordnung mit einer ersten Frequenz und einer zweiten Frequenz an den Patienten abzugeben;
    für jede der ersten Frequenz und der zweiten Frequenz die Intensität der Stimulationssignale zu erhöhen, bis eine Tachykardie-Herzratenreaktion bei dem Patienten detektiert wird;

für jede der ersten Frequenz und der zweiten Frequenz die Intensität der Stimulationssignale weiter zu erhöhen, bis eine Bradykardie-Herzratenreaktion bei dem Patienten detektiert wird; und

die therapeutischen Stimulationssignale an den Patienten mit einem von der ersten Frequenz und der zweiten Frequenz und mit einer Intensität innerhalb der neuralen Fulcrumzone der einen der ersten Frequenz und der zweiten Frequenz abzugeben.

6. Das Neurostimulationssystem nach Anspruch 5, wobei die Anweisungen die Steuerschaltung veranlassen, die Intensität der Stimulationssignale durch Erhöhen einer Ausgangsstromamplitude der Stimulationssignale zu erhöhen.

7. Das Neurostimulationssystem nach Anspruch 1, wobei die Anweisungen die Steuerschaltung veranlassen, ein oder mehrere interessierende Ereignisse zu extrahieren, die eine minimale Anzahl von gepaarten Maxima und Minima enthalten.

8. Das Neurostimulationssystem nach Anspruch 1, wobei die Anweisungen die Steuerschaltung veranlassen, ein oder mehrere Ereignisse von Interesse zu identifizieren, wobei jedes Ereignis von Interesse einer Zeitperiode entspricht, während der eine vordefinierte Anzahl von gepaarten Maxima und Minima nach einem anfänglichen Maximum auftritt.

9. Das Neurostimulationssystem nach Anspruch 1, wobei die Anweisungen die Steuerschaltung veranlassen:

eine Stimulationstherapie bei dem Patienten mit dem Neurostimulator zu initiieren;
ein Aktivitätsniveau des Patienten zu überwachen, um eine Änderung des Aktivitätsniveaus zu erkennen; und
ein oder mehrere Stimulationssignale in Reaktion auf die Änderung des Aktivitätsniveaus des Patienten zu modifizieren.

10. Das Neurostimulationssystem nach Anspruch 9, wobei die Anweisungen die Steuerschaltung veranlassen, das Aktivitätsniveau des Patienten durch Überwachung einer Herzrate des Patienten zu überwachen.

11. Das Neurostimulationssystem nach Anspruch 9, wobei die Anweisungen die Steuerschaltung veranlassen, das eine oder die mehreren Stimulationssignale durch Verringern einer Stimulationsintensität während einer Periode mit niedrigem Aktivitätsniveau und durch Erhöhen der Stimulationsintensität während einer Periode mit hohem Aktivitätsniveau zu modifizieren.

**Revendications**

1. Système de neurostimulation, comprenant :

un ensemble électrode (13, 14) ; et
un neurostimulateur (12 ; 900 ; 910, 920) couplé à l'ensemble électrode, le neurostimulateur comprenant :

un circuit de commande (902 ; 912) ; et
une mémoire (903 ; 913) fonctionnellement couplée au circuit de commande et comprenant des instructions qui, lorsqu'exécutées par le circuit de commande, amènent le circuit de commande à :

délivrer des signaux de stimulation à un patient par l'intermédiaire de l'ensemble électrode ;
accroître une intensité des signaux de stimulation jusqu'à ce qu'une réponse de fréquence cardiaque de tachycardie chez le patient est détectée ;
continuer à accroître l'intensité des signaux de stimulation jusqu'à ce qu'une réponse de fréquence cardiaque de bradycardie chez le patient est détectée ;
délivrer des signaux de stimulation thérapeutiques au patient à une intensité à l'intérieur d'une zone neurale pivot, la zone neurale pivot correspondant à une zone dans laquelle une réponse du patient à une stimulation est une réduction de 0 % à 5 % à partir d'une fréquence cardiaque de ligne de référence ;
calculer une médiane mobile (1012) d'un sous-ensemble de fréquences cardiaques sur une première période de temps ;
identifier des points maxima (1022) et minima (1021) appariés dans la médiane mobile ; et
identifier un ou plusieurs événements de récupération de la fréquence cardiaque, chaque événement de récupération de la fréquence cardiaque correspondant à une période de temps durant laquelle les points maxima et minima appariés satisfont un critère prédéterminé.

**2.** Système de neurostimulation selon la revendication 1, les signaux de stimulation comprenant une amplitude de courant de sortie, une largeur d'impulsion, une fréquence d'impulsion, et un cycle de charge.

**3.** Système de neurostimulation selon la revendication 2, les instructions amenant le circuit de commande à accroître l'intensité des signaux de stimulation en accroissant au moins l'une de l'amplitude de courant de sortie, de la largeur d'impulsion, de la fréquence d'impulsion, ou du cycle de charge des signaux de stimulation.

**4.** Système de neurostimulation selon la revendication 1, le système étant conçu pour délivrer les signaux de stimulation à un nerf vague du patient.

**5.** Système de neurostimulation selon la revendication 1, les instructions amenant en outre le circuit de commande à :

> délivrer les signaux de stimulation au patient par l'intermédiaire de l'ensemble électrode à une première fréquence et à une seconde fréquence ;
> pour chacune de la première fréquence et de la seconde fréquence, à accroître l'intensité des signaux de stimulation jusqu'à ce qu'une réponse de fréquence cardiaque de tachycardie chez le patient est détectée ;
> pour chacune de la première fréquence et de la seconde fréquence, à continuer d'accroître l'intensité des signaux de stimulation jusqu'à ce qu'une réponse de fréquence cardiaque de bradycardie chez le patient est détectée ; et
> à délivrer les signaux de stimulation thérapeutiques au patient à l'une de la première fréquence ou de la seconde fréquence et à une intensité à l'intérieur de la zone neurale pivot à l'une de la première fréquence ou de la seconde fréquence.

**6.** Système de neurostimulation selon la revendication 5, les instructions amenant le circuit de commande à accroître l'intensité des signaux de stimulation en accroissant une amplitude de courant de sortie des signaux de stimulation.

**7.** Système de neurostimulation selon la revendication 1, les instructions amenant le circuit de commande à extraire un ou plusieurs événements d'intérêt qui comprennent un nombre minimal de points maxima et minima appariés.

**8.** Système de neurostimulation selon la revendication 1, les instructions amenant le circuit de commande à identifier un ou plusieurs événements d'intérêt, chaque événement d'intérêt correspondant à une période de temps durant laquelle un nombre prédéfini de points maxima et minima appariés surviennent suite à un point maximum initial.

**9.** Système de neurostimulation selon la revendication 1, les instructions amenant le circuit de commande à :

> initier une thérapie de stimulation chez le patient avec le neurostimulateur ;
> surveiller un niveau d'activité du patient pour identifier un changement dans le niveau d'activité ; et
> à modifier un ou plusieurs signaux de stimulation en réponse au changement dans le niveau d'activité chez le patient.

**10.** Système de neurostimulation selon la revendication 9, les instructions amenant le circuit de commande à surveiller le niveau d'activité du patient en surveillant une fréquence cardiaque du patient.

**11.** Système de neurostimulation selon la revendication 9, les instructions amenant le circuit de commande à modifier les un ou plusieurs signaux de stimulation en réduisant une intensité de stimulation durant une période de niveau d'activité faible et en accroissant l'intensité de stimulation durant une période de niveau d'activité élevé.

**FIG. 1**

**FIG. 2A**

**FIG. 2B**

FIG. 3

FIG. 4

FIG. 5

FIG. 6

FIG. 7

**FIG. 8A**

**FIG. 8B**

## FIG. 8C

Control System
902

Memory
903

Physiological Sensor
904

Patient Input Sensor
905

Stimulation Subsystem
906

Implanted System
900

External Programmer
907

Patient Magnet
930

**FIG. 9A**

Control System
912

External Programmer
920

Memory
913

Physiological Sensor
914

Stimulation Subsystem
916

Implanted Medical Device
910

**FIG. 9B**

**FIG. 10A**

FIG. 10B

1100

| Start at i=0. | 1101 |

| i=i+1. | 1102 |

| Select the ith maxima point in the smoothed HR sequence. | 1103 |

| Include subsequent maxima points as long as they are decreasing by at least a given margin. Stop at the first non-decreasing maxima point. | 1104 |

| Include subsequent minima points as long as they are decreasing by at least a given margin. Stop at the first non decreasing minima point. | 1105 |

| The earliest stopping point in the previous steps 1104 and 1105 defines the end of current event. | 1106 |

| Repeat the previous steps 1102 thru 1106 until all maxima points have been examined. | 1107 |

| Retain all identified candidates with at least a minimum number of maxima points and a minimum number of valleys. | 1108 |

| Retain all identified candidates with a minimum and maximum change in HR. | 1109 |

**FIG. 11**

**FIG. 12**

Intrinsic Heart Rate Recovery (IHRR)

FIG. 13

FIG. 14

FIG. 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62114480
- US 2012095530 A
- US 8600505 B
- US 8571654 B
- US 8630709 B
- US 8577458 B
- US 13314119 B
- US 8918190 B
- US 8918191 B
- US 8923964 B

**Non-patent literature cited in the description**

- **J. HELLYER et al.** Autonomic Nerve Activity and Blood Pressure in Ambulatory Dogs. *Heart Rhythm,* February 2014, vol. 11 (2), 307-313